# EUROPÄISCHE PATENTANMELDUNG

(11) **EP 4 609 796 A1**
(43) Veröffentlichungstag der Anmeldung: **03.09.2025**
(21) Anmeldenummer: 25157446.3
(22) Anmeldetag: 12.02.2025
(51) Int. Cl.: A61B 6/46, A61G 11/00

(54) **VERFAHREN ZUM STEUERN EINER AKTORIK EINES WÄRMETHERAPIEGERÄTS**

(30) Priorität: 13.02.2024 DE 102024103998
(71) Anmelder: Löwenstein Medical Technology S.A., 2557 Luxembourg (LU)
(72) Erfinder: Stahl, Martin, 55432 Damscheid (DE); Martin, Johannes, 87527 Sonthofen (DE); Ruech, Carsten, 56321 Brey (DE)
(74) Vertreter: Löwenstein Medical IP

(57) **Zusammenfassung**

Ein Verfahren zum Steuern einer Aktorik (15) eines medizintechnischen Geräts (3) umfasst: Empfangen eines Sensorsignals (11), das eine mittels eines Sensors (9) erfasste Verformung eines Betätigungselements (7) bei dessen Betätigung mittels eines Fußes und/oder einer Hand anzeigt; Erzeugen eines Steuersignals (17) zum Steuern der Aktorik (15) unter Verwendung des Sensorsignals (11).

## Beschreibung

### Technisches Gebiet

Die Erfindung betrifft ein Verfahren zum Steuern einer Aktorik eines medizintechnischen Geräts. Darüber hinaus betrifft die Erfindung eine Signalverarbeitungseinrichtung zum Ausführen des Verfahrens, eine Bedienvorrichtung zum Bedienen eines medizintechnischen Gerätsund ein mit einer solchen Bedienvorrichtung ausgestattetes medizintechnisches Gerät.

### Stand der Technik

Ein medizintechnisches Gerät wie beispielsweise ein Wärmetherapiegerät, etwa in Form eines Inkubators oder eines Wärmebetts, kann mit einem oder mehreren Pedalen zum Steuern einer Aktorik des medizintechnischen Geräts ausgestattet sein. Bei einem solchen Pedal handelt es sich in der Regel um einen beweglich gelagerten Kipp- oder Druckschalter, der entsprechend anfällig für Funktionsbeeinträchtigungen infolge von mechanischem Verschleiß, Verschmutzung oder sonstigen Umwelteinflüssen sein kann.

### Offenbarung der Erfindung

Eine Aufgabe der Erfindung kann darin gesehen werden, ein verbessertes Verfahren zum Steuern einer Aktorik eines medizintechnischen Geräts zu schaffen. Eine weitere Aufgabe der Erfindung kann darin gesehen werden, eine Signalverarbeitungseinrichtung zum Ausführen eines solchen Verfahrens, eine entsprechende Bedienvorrichtung und ein entsprechendes medizintechnisches Gerät bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Verfahren zum Steuern einer Aktorik eines medizintechnischen Geräts. Das medizintechnische Gerät umfasst zusätzlich zur Aktorik eine Bedienvorrichtung zum Bedienen des medizintechnischen Geräts. Die Bedienvorrichtung umfasst ein mittels eines Fußes und/oder einer Hand (beispielsweise eines Fingers) betätigbares Betätigungselement und einen Sensor zum Erfassen einer Verformung des Betätigungselements. Das Verfahren umfasst: Empfangen eines Sensorsignals, das eine mittels des Sensors erfasste Verformung des Betätigungselements bei dessen Betätigung anzeigt; Erzeugen eines Steuersignals zum Steuern der Aktorik unter Verwendung des Sensorsignals.

Ein solches Verfahren ermöglicht es, die Aktorik ohne beweglich gelagerte Betätigungselemente zu steuern. Dadurch wird mechanischer Verschleiß verringert. Aufgrund des geringeren konstruktiven Aufwands im Vergleich zu Ausführungsformen mit beweglich gelagerten Betätigungselementen können zudem die Herstellungs-, Wartungs- und Reparaturkosten gesenkt werden.

Unter "Verformung" kann insbesondere eine (reversible) elastische Verformung verstanden werden. Die Verformung kann beispielsweise anhand einer (positiven oder negativen) Änderung von mindestens einer der folgenden vom Sensor erfassbaren elektrischen Größen, die direkt oder indirekt von der Verformung abhängen können, bestimmt werden: einer Spannung, einem Strom, einem Widerstand. Es ist möglich, dass der Sensor, beispielsweise in Form eines oder mehrerer Dehnungsmessstreifen, mit einer Vorspannung appliziert wird, sodass das Sensorsignal immer positiv ist, aber je nach Verformungsrichtung entweder gegen null tendiert oder größer wird.

Unter "Signal" wie in "Sensorsignal" oder "Steuersignal" kann ein analoges oder digitales elektrisches Signal verstanden werden. Das Steuersignal kann beispielsweise abhängig von einem Betrag, d. h. einer Intensität, und/oder einem Vorzeichen des Sensorsignals erzeugt werden.

Das Verfahren kann beispielsweise computerimplementiert sein.

Ein zweiter Aspekt der Erfindung betrifft eine Signalverarbeitungseinrichtung. Die Signalverarbeitungseinrichtung umfasst Mittel, die konfiguriert sind, um das vor- und nachstehend beschriebene Verfahren auszuführen.

Die Mittel können Hard- und/oder Softwaremodule umfassen. Insbesondere können die Mittel einen Prozessor umfassen, der konfiguriert ist, um das Verfahren (durch Ausführen von Befehlen eines entsprechenden Computerprogramms) auszuführen. Zusätzlich können die Mittel einen Speicher und/oder eine Datenkommunikationsschnittstelle zur drahtlosen und/oder drahtgebundenen Datenkommunikation mit Peripheriegeräten umfassen. Alternativ kann die Signalverarbeitungseinrichtung ausschließlich als Hardware, beispielsweise in Form eines ASIC- oder FPGA-Bausteins, implementiert sein.

Beispielsweise kann die Signalverarbeitungseinrichtung mindestens eines der folgenden Mittel umfassen: einen Verstärker zum Verstärken eines analogen Sensorsignals; einen Analog-digital-Wandler zum Umwandeln eines (gegebenenfalls verstärkten) analogen Sensorsignals in ein digitales Sensorsignal; eine Signalanalyseeinheit zum Analysieren eines (gegebenenfalls verstärkten) analogen Sensorsignals, beispielsweise durch Filtern und/oder Fourier-Transformation; eine Verarbeitungseinheit zum Weiterverarbeiten eines digitalen Sensorsignals und/oder eines (gegebenenfalls verstärkten und/oder durch eine entsprechende Signalanalyse vorverarbeiteten) analogen Sensorsignals.

Es ist möglich, dass mindestens eines der vorgenannten Mittel der Signalverarbeitungseinrichtung als ein Hard- und/oder Softwaremodul des Sensors implementiert ist (und umgekehrt).

Es wird darauf hingewiesen, dass Merkmale des vor- und nachstehend beschriebenen Verfahrens auch Merkmale der Signalverarbeitungseinrichtung sein können (und umgekehrt).

Ein dritter Aspekt der Erfindung betrifft eine Bedienvorrichtung zum Bedienen eines medizintechnischen Geräts. Die Bedienvorrichtung umfasst: ein mittels eines Fußes und/oder einer Hand betätigbares Betätigungselement; einen Sensor, der konfiguriert ist, um eine Verformung des Betätigungselements bei dessen Betätigung zu erfassen und ein die erfasste Verformung des Betätigungselements anzeigendes Sensorsignal zu erzeugen; eine Signalverarbeitungseinrichtung, wie sie vor- und nachstehend beschrieben wird.

Eine solche Bedienvorrichtung hat den Vorteil, dass sie ohne beweglich gelagerte Betätigungselemente auskommt. Die Bedienvorrichtung ist daher entsprechend verschleißfest und robust gegenüber Umwelteinflüssen wie z. B. mechanischen Erschütterungen, Schmutz, Nässe oder Feuchtigkeit. Zudem erfordert eine solche Bedienvorrichtung einen geringeren konstruktiven Aufwand als Ausführungsformen mit beweglich gelagerten Betätigungselementen. Dies kann die Herstellung, Wartung und Reparatur vereinfachen und somit die entsprechenden Kosten senken.

Der Sensor kann beispielsweise ein analoger oder inkrementeller Wegsensor (z. B. in Form eines Dehnungsmessstreifens), ein optischer Sensor (z. B. in Form eines Faser-Bragg-Gitters) oder eine Kombination aus mindestens zwei dieser Beispiele sein.

Ein vierter Aspekt der Erfindung betrifft ein medizintechnisches Gerät. Das medizintechnische Gerät umfasst eine Aktorik und eine Bedienvorrichtung, wie sie vor- und nachstehend beschrieben wird.

Unter "medizintechnisches Gerät" kann beispielsweise ein Wärmetherapiegerät zum Durchführen einer Wärmetherapie mit einer Liegefläche für einen Patienten verstanden werden. Ein solches Wärmetherapiegerät kann insbesondere als ein Wärmebett, ein Inkubator oder eine Reanimationseinheit - beispielsweise jeweils für ein Früh- oder Neugeborenes, einen Säugling oder ein Baby - ausgeführt sein. Alternativ kann es sich bei dem medizintechnischen Gerät um einen Operations- oder Arbeitstisch, ein Krankenbett, eine Untersuchungsliege, einen Behandlungsstuhl, ein Diagnosegerät (beispielsweise einen Computer- oder Magnetresonanztomografen) oder ein Röntgengerät handeln.

Die Aktorik kann durch einen oder mehrere mittels des Steuersignals steuerbare Aktoren gebildet sein. Ein solcher Aktor kann beispielsweise ein Elektromotor, ein Elektromagnet oder ein elektromechanisches Ventil sein.

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann die Bedienvorrichtung ferner eine Rückmeldungseinrichtung zum Erzeugen einer akustischen und/oder optischen und/oder haptischen Rückmeldung für einen (menschlichen) Anwender oder Bediener des medizintechnischen Geräts umfassen.

Die Rückmeldungseinrichtung kann beispielsweise mindestens eine der folgenden Komponenten umfassen: eine Lichtquelle (z. B. in Form mindestens einer Leuchtdiode, mindestens einer Glühbirne oder mindestens eines beispielsweise länglichen Lichtwellenleiters) zum Erzeugen der optischen Rückmeldung; ein Display zum Erzeugen der optischen Rückmeldung; einen Lautsprecher zum Erzeugen der akustischen Rückmeldung (z. B. eines Signaltons, eines Geräuschs oder eines Sprachhinweises); einen Vibrationsgeber zum Versetzen des Betätigungselements in Vibrationen (z. B. in Form eines speziellen Elektromotors oder eines Lautsprechers, der mit einer entsprechend niedrigen Frequenz betrieben werden kann).

Die Lichtquelle kann beispielsweise ausgebildet sein, um zum Erzeugen der optischen Rückmeldung das medizintechnische Gerät selbst und/oder einen Boden, auf dem das medizintechnische Gerät im betriebsfähigen Zustand steht, in geeigneter Weise zu beleuchten und/oder die Helligkeit und/oder Farbe des ausgesandten Lichts in geeigneter Weise zu variieren.

Gemäß einer Ausführungsform kann das Verfahren ferner umfassen: Erzeugen eines zusätzlichen Steuersignals zum Steuern der Rückmeldungseinrichtung unter Verwendung des Sensorsignals und/oder des Steuersignals.

Es ist möglich, dass die Rückmeldungseinrichtung als Reaktion auf das Betätigen des Betätigungselements für eine bestimmte Dauer aktiviert wird, beispielsweise so lange, wie das Sensorsignal (mit ausreichender Stärke) empfangen wird und/oder das Steuersignal erzeugt wird. Unter "Aktivieren" kann beispielsweise ein Einschalten oder ein abwechselndes Ein- und Ausschalten verstanden werden.

Mithilfe einer solchen zusätzlichen Rückmeldung kann der Bedienkomfort weiter verbessert werden.

Gemäß einer Ausführungsform kann die erfasste Verformung einen erfassten Verformungsgrad umfassen, wobei das Steuersignal abhängig vom erfassten Verformungsgrad erzeugt werden kann. Der erfasste Verformungsgrad kann beispielsweise ein Betrag (eines aktuellen Amplitudenwerts) des Sensorsignals sein. Alternativ kann der erfasste Verformungsgrad ein Prozentwert sein. Dabei kann 0 Prozent für einen (beispielsweise unverformten) Ausgangszustand des Betätigungselements und 100 Prozent für eine zulässige maximale Verformung des Betätigungselements stehen. Dies ermöglicht es, die Aktorik abhängig von der jeweiligen Betätigungskraft, mit der das Betätigungselement gerade betätigt (d. h. verformt) wird, anzusteuern. Dazu kann beispielsweise ein aktueller Amplitudenwert einem bestimmten Einstellungswert aus mehreren möglichen Einstellungswerten für mindestens einen Steuerparameter zum Steuern der Aktorik - z. B. eine Beschleunigung oder Geschwindigkeit, mit der ein bestimmter Aktor der Aktorik bewegt werden soll - zugeordnet und auf den jeweiligen Steuerparameter angewandt werden.

Gemäß einer Ausführungsform kann die erfasste Verformung eine erfasste Verformungsrichtung umfassen, wobei das Steuersignal abhängig von der erfassten Verformungsrichtung erzeugt werden kann. Die erfasste Verformungsrichtung kann beispielsweise ein Vorzeichen (eines aktuellen Amplitudenwerts) des Sensorsignals sein. Dies ermöglicht es, die Aktorik abhängig von der jeweiligen Betätigungsrichtung, in der das Betätigungselement gerade betätigt (d. h. verformt) wird, anzusteuern. Dazu kann beispielsweise anhand des Vorzeichens des aktuellen Amplitudenwerts ein Vorzeichen für den aktuellen Einstellungswert des mindestens einen Steuerparameters bestimmt und auf den jeweiligen Steuerparameter angewandt werden. Insbesondere kann das Betätigungselement in einander entgegengesetzten Richtungen betätigbar sein.

Alternativ oder zusätzlich kann das zusätzliche Steuersignal (siehe weiter oben) abhängig vom erfassten Verformungsgrad und/oder von der erfassten Verformungsrichtung erzeugt werden. Dies ermöglicht es, die Rückmeldung in ihrer Art und/oder Intensität abhängig von der jeweiligen Betätigungskraft und/oder -richtung zu variieren, was sich vorteilhaft auf den Bedienkomfort auswirken kann.

Gemäß einer Ausführungsform kann der Sensor einen mit zumindest einem Abschnitt des Betätigungselements mechanisch gekoppelten Dehnungsmessstreifen umfassen. Unter "Dehnungsmessstreifen" kann allgemein ein Dehnungssensor zum Umwandeln einer mechanischen Verformung in ein elektrisches (Sensor-)Signal verstanden werden. Der Dehnungsmessstreifen kann so ausgebildet sein, dass sich ein elektrischer Widerstand eines die Anschlüsse des Dehnungsmessstreifens elektrisch leitfähig miteinander verbindenden Materials abhängig von dessen Verformung ändert. Ein solcher Dehnungsmessstreifen kann beispielsweise als flächiges und/oder längliches Element (z. B. in Form eines Streifens oder einer Folie) ausgebildet sein. Möglich sind aber auch andere Ausführungsformen des Dehnungsmessstreifens (der Dehnungsmessstreifen muss nicht zwingend als "Streifen" ausgebildet sein). Es ist zweckmäßig, wenn der Dehnungsmessstreifen auf einen Abschnitt des Betätigungselements aufgebracht ist, der bei dessen Betätigung stärker als der restliche Abschnitt des Betätigungselements gedehnt und/oder gestaucht wird.

Gemäß einer Ausführungsform kann das Sensorsignal eine an den Anschlüssen des Dehnungsmessstreifens anliegende elektrische Spannung und/oder einen zwischen den Anschlüssen des Dehnungsmessstreifens fließenden elektrischen Strom als die erfasste Verformung anzeigen. Dabei kann ein Betrag der Spannung bzw. des Stroms vom jeweiligen Verformungsgrad des Betätigungselements und/oder kann ein Vorzeichen der Spannung bzw. des Stroms von der jeweiligen Verformungsrichtung des Betätigungselements abhängen.

Gemäß einer Ausführungsform kann eine Abweichung einer Amplitude der Spannung und/oder des Stroms von einem Schwellenwert bestimmt werden und das Steuersignal abhängig von der Abweichung erzeugt werden. Die Abweichung kann beispielsweise durch Subtrahieren eines jeweiligen Werts der Amplitude vom Schwellenwert bestimmt werden. Unter "Schwellenwert" kann allgemein ein (vordefinierter) Referenzwert verstanden werden. Der Schwellenwert kann fest vorgegeben oder variierbar sein, etwa um eine Feinjustierung der Bedienvorrichtung zu ermöglichen. Das Steuersignal kann insbesondere dann erzeugt werden, wenn die Amplitude den Schwellenwert erreicht, d. h. die Abweichung gegen null geht. Auf diese Weise können Fehlbedienungen vermieden werden, etwa wenn das Betätigungselement versehentlich leicht berührt wird.

Gemäß einer Ausführungsform kann der Sensor ein erstes Sensorelement und ein zweites Sensorelement umfassen. Das erste Sensorelement kann konfiguriert sein, um eine Verformung eines ersten Abschnitts des Betätigungselements bei dessen Betätigung zu erfassen und ein die erfasste Verformung des ersten Abschnitts anzeigendes erstes Sensorsignal zu erzeugen. Das zweite Sensorelement kann konfiguriert sein, um eine Verformung eines vom ersten Abschnitt abweichenden zweiten Abschnitts des Betätigungselements bei dessen Betätigung zu erfassen und ein die erfasste Verformung des zweiten Abschnitts anzeigendes zweites Sensorsignal zu erzeugen. In diesem Fall kann die Signalverarbeitungseinrichtung der Bedienvorrichtung konfiguriert sein, um das Steuersignal unter Verwendung des ersten Sensorsignals und/oder des zweiten Sensorsignals zu erzeugen. Anders ausgedrückt kann das Steuersignal wahlweise aus dem ersten Sensorsignal, dem zweiten Sensorsignal oder aus beiden Sensorsignalen erzeugt werden.

Gemäß einer Ausführungsform kann das Empfangen des Sensorsignals umfassen: Empfangen eines ersten Sensorsignals, das eine mittels des ersten Sensorelements erfasste Verformung des ersten Abschnitts des Betätigungselements bei dessen Betätigung anzeigt; Empfangen eines zweiten Sensorsignals, das eine mittels des zweiten Sensorelements erfasste Verformung des zweiten Abschnitts des Betätigungselements bei dessen Betätigung anzeigt. Dementsprechend kann das Steuersignal unter Verwendung des ersten Sensorsignals und/oder des zweiten Sensorsignals erzeugt werden. Dies ermöglicht eine genauere Messung der Verformung im Vergleich zu einer Ausführungsform mit nur einem Sensorelement. Ein weiterer Vorteil ist, dass die Aktorik auch dann noch gesteuert werden kann, wenn eines der Sensorelemente ausfällt.

Gemäß einer Ausführungsform kann das erste Sensorelement ein mit dem ersten Abschnitt mechanisch gekoppelter erster Dehnungsmessstreifen sein. Dementsprechend kann das erste Sensorsignal eine an den Anschlüssen des ersten Dehnungsmessstreifens anliegende elektrische Spannung und/oder einen zwischen den Anschlüssen des ersten Dehnungsmessstreifens fließenden elektrischen Strom anzeigen.

Gemäß einer Ausführungsform kann das zweite Sensorelement ein mit dem zweiten Abschnitt mechanisch gekoppelter zweiter Dehnungsmessstreifen sein. Dementsprechend kann das zweite Sensorsignal eine an den Anschlüssen des zweiten Dehnungsmessstreifens anliegende elektrische Spannung und/oder einen zwischen den Anschlüssen des zweiten Dehnungsmessstreifens fließenden elektrischen Strom anzeigen.

Der erste und der zweite Dehnungsmessstreifen können sich in ihrer Position und/oder Orientierung gegenüber dem Betätigungselement voneinander unterscheiden. Dabei können die Längsachsen des ersten und des zweiten Dehnungsmessstreifens parallel oder schräg zueinander ausgerichtet sein. Beispielsweise können die zueinander schräg ausgerichteten Längsachsen einen Winkel von 90 Grad oder weniger, von 60 Grad oder weniger oder von 30 Grad oder weniger einschließen.

Gemäß einer Ausführungsform können die Anschlüsse des ersten Dehnungsmessstreifens mit den Anschlüssen des zweiten Dehnungsmessstreifens über eine Brückenschaltung verschaltet sein, um ein drittes Sensorsignal aus dem ersten Sensorsignal und dem zweiten Sensorsignal zu erzeugen. In diesem Fall kann das Steuersignal unter Verwendung des dritten Sensorsignals erzeugt werden. Dies kann die Zuverlässigkeit und/oder Genauigkeit des Verfahrens weiter verbessern. Das dritte Sensorsignal kann beispielsweise eine größere Amplitude als das erste Sensorsignal und/oder das zweite Sensorsignal haben. Die Brückenschaltung kann beispielsweise eine Vollbrücke, eine Halbbrücke, eine Viertelbrücke oder eine Kombination aus mindestens zwei dieser Beispiele umfassen.

Gemäß einer Ausführungsform kann die Bedienvorrichtung ferner ein mittels eines Fußes und/oder einer Hand betätigbares weiteres Betätigungselement und einen weiteren Sensor zum Erfassen einer Verformung des weiteren Betätigungselements umfassen. Das Betätigungselement und das weitere Betätigungselement können beispielsweise unabhängig voneinander betätigbar sein.

Gemäß einer Ausführungsform kann das Verfahren ferner umfassen: Empfangen eines weiteren Sensorsignals, das eine mittels des weiteren Sensors erfasste Verformung des weiteren Betätigungselements bei dessen Betätigung anzeigt; Erzeugen eines weiteren Steuersignals zum Steuern der Aktorik unter Verwendung des weiteren Sensorsignals oder unter Verwendung des Sensorsignals und des weiteren Sensorsignals. Somit können Fehlbedienungen vermieden werden, beispielsweise indem eine versehentliche gleichzeitige Betätigung der verschiedenen Betätigungselemente erkannt wird. Zu diesem Zweck kann beispielsweise eine Abweichung des Sensorsignals vom weiteren Sensorsignal bestimmt werden, wobei das Steuersignal abhängig von der Abweichung erzeugt werden kann. Es ist möglich, dass mit dem weiteren Steuersignal die gleiche Einrichtung der Aktorik wie mit dem Steuersignal angesteuert werden kann. Alternativ ist es möglich, dass mit dem weiteren Steuersignal eine andere Einrichtung der Aktorik als mit dem Steuersignal angesteuert werden kann.

Gemäß einer Ausführungsform kann das Betätigungselement plattenartig und/oder aus Metall ausgebildet sein. Ein solches Betätigungselement ist besonders robust und/oder kann besonders einfach hergestellt werden.

Gemäß einer Ausführungsform kann das Betätigungselement an seinem ersten Ende starr mit einem Befestigungsabschnitt des medizintechnischen Geräts verbindbar sein und durch Aufbringen einer definierten Biegekraft auf sein zweites, freies Ende betätigbar sein. Das Betätigungselement kann an seinem ersten Ende beispielsweise durch Schrauben, Schweißen, Löten, Kleben oder eine Kombination aus mindestens zwei dieser Verbindungsmethoden starr mit dem Befestigungsabschnitt verbindbar sein. Unter "Befestigungsabschnitt" kann insbesondere ein Abschnitt einer tragenden Struktur des medizintechnischen Geräts, beispielsweise eines (Fahr-)Gestells, verstanden werden. Ein solcher Befestigungsabschnitt ist in der Regel besonders steif. Dies hat die Wirkung, dass sich beim Betätigen des Betätigungselements vor allem das Betätigungselement und weniger der Befestigungsabschnitt verformt. Dies ermöglicht zum einen eine besonders feinfühlige Betätigung. Zum anderen können somit größere Ungenauigkeiten beim Erfassen der Verformung des Betätigungselements vermieden werden.

Gemäß einer Ausführungsform kann mindestens eine (elektrische und/oder elektronische) Komponente der Bedienvorrichtung auf einer Leiterplatte angeordnet sein, wobei die Leiterplatte am Betätigungselement befestigt sein kann. Aufgrund der Befestigung mittels der Leiterplatte kann die betreffende Komponente oder können die betreffenden Komponenten in einem einzigen Schritt zusammen mit dem Betätigungselement montiert bzw. demontiert werden, was die Montage bzw. Demontage vereinfacht. Insbesondere kann mindestens eine der folgenden Komponenten der Bedienvorrichtung auf der Leiterplatte angeordnet sein: der Sensor (oder mindestens eine Komponente des Sensors), die Signalverarbeitungseinrichtung (oder mindestens eine Komponente der Signalverarbeitungseinrichtung), die Rückmeldungseinrichtung (oder mindestens eine Komponente der Rückmeldungseinrichtung), ein Anschluss für eine Stromversorgung.

Zusätzlich oder alternativ kann mindestens eine (elektrische und/oder elektronische) Komponente der Bedienvorrichtung auf einer externen Leiterplatte angeordnet sein, wobei die externe Leiterplatte an einem vom Betätigungselement abweichenden Abschnitt des medizintechnischen Geräts, beispielsweise an dessen (Fahr-)Gestell, befestigbar sein kann.

Gemäß einer Ausführungsform kann die Leiterplatte mittels mindestens eines Abstandhalters am Betätigungselement befestigt sein, sodass die Leiterplatte und das Betätigungselement durch einen Spalt voneinander getrennt sind. In diesem Fall kann die mindestens eine auf der Leiterplatte angeordnete Komponente im Spalt angeordnet sein. Auf diese Weise kann die betreffende Komponente oder können die betreffenden Komponenten wirksam vor Umwelteinflüssen geschützt werden.

Gemäß einer Ausführungsform kann der Spalt zusätzlich abgedichtet sein, um die mindestens eine im Spalt angeordnete Komponente vor Umwelteinflüssen wie z. B. Schmutz, Feuchtigkeit, Nässe, Erschütterungen oder elektromagnetischer Strahlung zu schützen. Der Spalt kann insbesondere fluiddicht abgedichtet und/oder zumindest teilweise mit einem geeigneten Dichtmaterial ausgefüllt sein, beispielsweise in Form eines oder mehrerer Dichtringe und/oder einer Vergussmasse (z. B. aus Kunstharz und/oder Silikon).

Gemäß einer Ausführungsform kann die mindestens eine auf der Leiterplatte (und gegebenenfalls im Spalt) angeordnete Komponente mindestens eine (elektrische und/oder elektronische) Komponente der Rückmeldungseinrichtung umfassen, beispielsweise mindestens eine der folgenden Komponenten der Rückmeldungseinrichtung (siehe weiter oben): die Lichtquelle, das Display, den Lautsprecher, den Vibrationsgeber. Ist die Lichtquelle im Spalt angeordnet und ist der Spalt zusätzlich abgedichtet, so ist es zweckmäßig, wenn das Dichtmaterial lichtdurchlässig ist, sodass das von der Lichtquelle ausgesandte Licht von außen für den Anwender des medizintechnischen Geräts sichtbar ist.

Gemäß einer Ausführungsform kann das medizintechnische Gerät ein Wärmetherapiergerät sein. Das Wärmetherapiergerät kann beispielsweise als ein Wärmebett, ein Inkubator, eine Reanimationseinheit oder eine Kombination von mindestens zwei dieser Beispiele ausgeführt sein (z. B. jeweils für ein Früh- oder Neugeborenes, einen Säugling oder ein Baby).

Gemäß einer Ausführungsform kann das medizintechnische Gerät ferner eine Liegefläche für einen Patienten umfassen. In diesem Fall kann die Aktorik eine durch die Signalverarbeitungseinrichtung der Bedienvorrichtung steuerbare Liegeflächenverstelleinrichtung zum Bewegen der Liegefläche umfassen, beispielsweise um deren Position und/oder Orientierung relativ zum Boden, auf dem das medizintechnische Gerät steht, zu verstellen. Unter "Liegefläche" kann beispielsweise ein (beheizbares) Liegedeck verstanden werden. Die Liegeflächenverstelleinrichtung kann beispielsweise eine oder mehrere Hubsäulen und/oder eine wippenartige Lagerung der Liegefläche umfassen.

Gemäß einer Ausführungsform kann das medizintechnische Gerät ferner eine Inkubatorkammer zum Aufnehmen eines Früh- oder Neugeborenen umfassen. In diesem Fall kann die Aktorik mindestens eine der folgenden Einrichtungen, die durch die Signalverarbeitungseinrichtung der Bedienvorrichtung steuerbar sein können, umfassen: eine Kammerverstelleinrichtung zum Bewegen der Inkubatorkammer, beispielsweise um deren Position und/oder Orientierung relativ zum Boden, auf dem das medizintechnische Gerät steht, zu verstellen; eine Haubenverstelleinrichtung zum Bewegen einer Haube der Inkubatorkammer, beispielsweise um die Inkubatorkammer mittels der Haube zu öffnen und/oder zu schließen. Die Kammerverstelleinrichtung kann beispielsweise eine oder mehrere Hubsäulen zum Verstellen der Inkubatorkammer umfassen. Die Inkubatorkammer kann beheizbar sein. Zusätzlich oder alternativ kann die Luftfeuchtigkeit innerhalb der Inkubatorkammer regelbar sein.

Gemäß einer Ausführungsform kann das medizintechnische Gerät ferner ein Fahrgestell mit mehreren Rollen zum Verfahren des medizintechnischen Geräts umfassen. In diesem Fall kann die Aktorik mindestens eine der folgenden Einrichtungen, die durch die Signalverarbeitungseinrichtung der Bedienvorrichtung steuerbar sein können, umfassen:
eine Antriebseinrichtung zum Antreiben von mindestens einer der Rollen; eine Bremseinrichtung zum Bremsen von mindestens einer der Rollen (beispielsweise in Form einer elektrischen Parkbremse); eine Lenkeinrichtung zum Lenken von mindestens einer der Rollen.

Unter "Bremsen" und/oder "Lenken" kann auch ein Arretieren in einer oder mehreren Richtungen verstanden werden.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt eine Bedienvorrichtung gemäß einer Ausführungsform der Erfindung.
Fig. 2 zeigt ein Betätigungselement aus Fig. 1 von unten.
Fig. 3 zeigt ein medizintechnisches Gerät gemäß einer Ausführungsform der Erfindung.
Fig. 4 zeigt einen Abschnitt eines Fahrgestells eines medizintechnischen Geräts gemäß einer Ausführungsform der Erfindung.
Fig. 5 zeigt einen Abschnitt des Fahrgestells aus Fig. 4 von unten.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

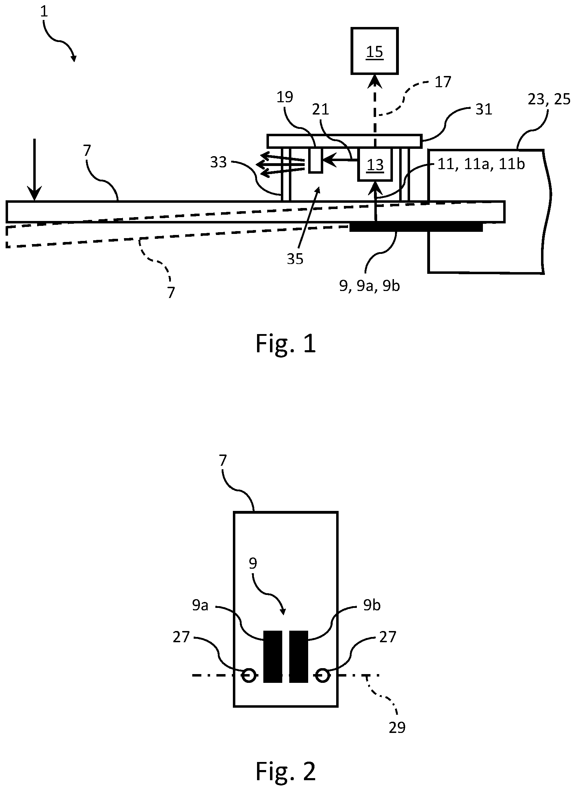

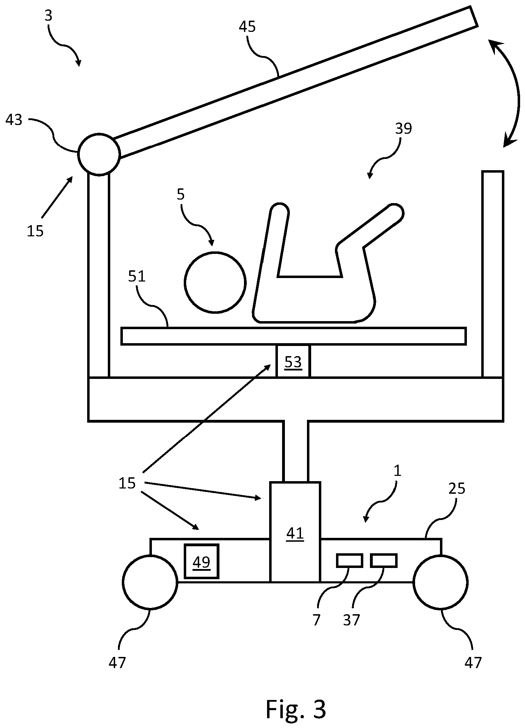

Fig. 1 zeigt eine Bedienvorrichtung 1 zum Bedienen eines medizintechnischen Geräts 3, hier eines Inkubators 3 für ein Früh- oder Neugeborenes 5 (siehe Fig. 3). Die Bedienvorrichtung 1 umfasst ein mittels eines Fußes und/oder einer Hand (beispielsweise eines Fingers) betätigbares Betätigungselement 7, einen Sensor 9, der konfiguriert ist, um eine (überwiegend elastische) Verformung des Betätigungselements 7 bei dessen Betätigung zu erfassen und ein die erfasste Verformung des Betätigungselements 7 anzeigendes analoges oder digitales elektrisches Sensorsignal 11 zu erzeugen, sowie eine entsprechende Signalverarbeitungseinrichtung 13.

Die Signalverarbeitungseinrichtung 13 umfasst Mittel, die konfiguriert sind, um das folgende Verfahren zum Steuern einer Aktorik 15 des Inkubators 3 auszuführen.

In einem ersten Schritt des Verfahrens wird das Sensorsignal 11 in der Signalverarbeitungseinrichtung 13 empfangen. Anschließend wird in einem zweiten Schritt des Verfahrens ein Steuersignal 17 zum Steuern der Aktorik 15 erzeugt.

Die Aktorik 15 kann beispielsweise mindestens einen der folgenden durch die Signalverarbeitungseinrichtung 13 steuerbaren Aktoren umfassen: einen Elektromotor, einen Elektromagnet, ein elektromechanisches Ventil.

Die Mittel der Signalverarbeitungseinrichtung 13 können beispielsweise einen Prozessor und einen Speicher umfassen. In diesem Fall kann der Prozessor konfiguriert sein, um das Verfahren durch Ausführen eines im Speicher gespeicherten Computerprogramms auszuführen.

Zusätzlich kann die Bedienvorrichtung 1 eine Rückmeldungseinrichtung 19 zum Erzeugen einer akustischen und/oder optischen und/oder haptischen Rückmeldung für einen Anwender des Inkubators 3 umfassen. In diesem Fall kann in einem zusätzlichen Schritt des Verfahrens ein zusätzliches Steuersignal 21 zum Steuern der Rückmeldungseinrichtung 19 unter Verwendung des Sensorsignals 11 und/oder des Steuersignals 17 erzeugt werden.

Die Rückmeldungseinrichtung 19 kann beispielsweise mindestens eine der folgenden Komponenten umfassen: eine Lichtquelle (z. B. in Form mindestens einer Leuchtdiode, mindestens einer Glühbirne oder mindestens eines beispielsweise länglichen Lichtwellenleiters) zum Erzeugen der optischen Rückmeldung; ein Display zum Erzeugen der optischen Rückmeldung; einen Lautsprecher zum Erzeugen der akustischen Rückmeldung (z. B. eines Signaltons, eines Geräuschs oder eines Sprachhinweises); einen Vibrationsgeber zum Versetzen des Betätigungselements 7 in Vibrationen (z. B. in Form eines speziellen Elektromotors oder eines Lautsprechers, der mit einer entsprechend niedrigen Frequenz betrieben werden kann). Die Lichtquelle kann beispielsweise ausgebildet sein, um zum Erzeugen der optischen Rückmeldung einen Boden, auf dem der Inkubator 3 im betriebsfähigen Zustand steht, mit einem bestimmten Muster zu beleuchten und/oder die Helligkeit und/oder Farbe des ausgesandten Lichts in geeigneter Weise zu variieren.

Das zusätzliche Steuersignal 21 kann beispielsweise als (unmittelbare) Reaktion auf das Empfangen des Sensorsignals 11 und/oder auf das Erzeugen des Steuersignals 17 erzeugt werden. Es ist möglich, dass das zusätzliche Steuersignal 21 so lange erzeugt wird, wie das Sensorsignal 11 (mit ausreichender Stärke) empfangen wird und/oder das Steuersignal 17 erzeugt wird.

Das Sensorsignal 11 kann einen erfassten Verformungsgrad und/oder eine erfasste Verformungsrichtung (in Fig. 1 angedeutet durch einen nach unten gerichteten senkrechten Pfeil) anzeigen. Dementsprechend kann das Steuersignal 17 abhängig vom erfassten Verformungsgrad und/oder von der erfassten Verformungsrichtung erzeugt werden. Dies ermöglicht es, die Aktorik 15 abhängig von der jeweiligen Betätigungskraft und/oder -richtung zu steuern.

Der erfasste Verformungsgrad kann beispielsweise einem aktuellen Betrag des Sensorsignals 11, d. h. dessen aktueller Intensität, entsprechen. Alternativ kann der erfasste Verformungsgrad ein Prozentwert zwischen 0 (für einen beispielsweise unverformten Ausgangszustand des Betätigungselements 7) und 1 (für eine zulässige maximale Verformung des Betätigungselements 7) sein. Die erfasste Verformungsrichtung kann beispielsweise einem aktuellen positiven oder negativen Vorzeichen des Sensorsignals 11 entsprechen, wobei jedes der Vorzeichen eine von zwei einander entgegengesetzten Verformungsrichtungen anzeigen kann.

Das Steuersignal 17 kann beispielsweise abhängig von einer Abweichung einer Amplitude des Sensorsignals 11 von einem vorgegebenen festen oder variierbaren Schwellenwert erzeugt werden, insbesondere nur dann, wenn die Amplitude den Schwellenwert erreicht oder überschreitet. Auf diese Weise können Fehlbedienungen vermieden werden, etwa wenn das Betätigungselement 7 versehentlich leicht gedrückt wird.

Wie in Fig. 2 zu sehen, kann der Sensor 9 ein erstes Sensorelement 9a zum Erfassen einer Verformung eines ersten Abschnitts des Betätigungselements 7 und ein zweites Sensorelement 9b zum Erfassen einer Verformung eines vom ersten Abschnitt abweichenden zweiten Abschnitts des Betätigungselements 7 umfassen. Der Sensor 9 kann auch mehr als zwei solcher Sensorelemente umfassen.

Dementsprechend können in der Signalverarbeitungseinrichtung 13 ein erstes Sensorsignal 11a, das eine mittels des ersten Sensorelements 9a erfasste Verformung des ersten Abschnitts des Betätigungselements 7 bei dessen Betätigung anzeigt, und ein zweites Sensorsignal 11b, das eine mittels des zweiten Sensorelements 9b erfasste Verformung des zweiten Abschnitts des Betätigungselements 7 bei dessen Betätigung anzeigt, empfangen werden. Das Steuersignal 17 kann dann unter Verwendung des ersten Sensorsignals 11a und/oder des zweiten Sensorsignals 11b erzeugt werden. Dies ermöglicht eine genauere Messung der Verformung im Vergleich zu einer Ausführungsform mit nur einem Sensorelement. Ein weiterer Vorteil ist, dass die Aktorik 15 auch dann noch gesteuert werden kann, wenn eines der Sensorelemente 9a, 9b ausfällt.

Die Sensorelemente 9a, 9b können jeweils als ein mit dem jeweiligen Abschnitt des Betätigungselements 7 mechanisch gekoppelter (beispielsweise aufgeklebter) Dehnungsmessstreifen ausgebildet sein.

Dementsprechend können die Sensorsignale 11a, 11b jeweils eine an den Anschlüssen des jeweiligen Dehnungsmessstreifens anliegende elektrische Spannung und/oder einen zwischen den Anschlüssen des jeweiligen Dehnungsmessstreifens fließenden elektrischen Strom anzeigen.

Die verschiedenen Dehnungsmessstreifen können sich in ihrer Position und/oder Orientierung gegenüber dem Betätigungselement 7 voneinander unterscheiden. Dabei können die jeweiligen Längsachsen der Dehnungsmessstreifen - wie in Fig. 2 beispielhaft gezeigt - parallel oder schräg zueinander ausgerichtet sein. Beispielsweise können die zueinander schräg ausgerichteten Längsachsen einen Winkel von 90 Grad oder weniger, von 60 Grad oder weniger oder von 30 Grad oder weniger einschließen.

Optional können die Anschlüsse der verschiedenen Dehnungsmessstreifen über eine Brückenschaltung miteinander verschaltet sein. Die Brückenschaltung kann konfiguriert sein, um ein drittes Sensorsignal aus dem ersten Sensorsignal 11a und dem zweiten Sensorsignal 11b zu erzeugen. Das dritte Sensorsignal kann beispielsweise eine größere Amplitude als jedes der beiden anderen Sensorsignale 11a, 11b haben. Die Brückenschaltung kann beispielsweise eine Vollbrücke, eine Halbbrücke, eine Viertelbrücke oder eine Kombination aus mindestens zwei dieser Beispiele umfassen. Das Steuersignal 17 kann dann unter Verwendung des dritten Sensorsignals erzeugt werden. Dies kann die Zuverlässigkeit und/oder Genauigkeit des Verfahrens weiter verbessern.

In dem in Fig. 1 und Fig. 2 gezeigten Beispiel sind die Sensorelemente 9a, 9b jeweils auf eine Unterseite des plattenartigen Betätigungselements 7 aufgebracht. Dort wird das Betätigungselement 7 bei dessen Betätigung besonders stark gestaucht, d. h. auf Druck beansprucht. Möglich sind auch andere Stellen, beispielsweise eine Stelle auf einer der Unterseite gegenüberliegenden Oberseite des Betätigungselements 7. Dort wird das Betätigungselement 7 bei dessen Betätigung besonders stark gedehnt, d. h. auf Zug beansprucht.

Wie in Fig. 1 gezeigt, kann das Betätigungselement 7 an seinem ersten Ende starr mit einem Befestigungsabschnitt 23 des Inkubators 3 verbunden sein. Das Betätigungselement 7 kann dann durch Aufbringen einer vertikalen Kraft auf sein freies, zweites Ende betätigt, d. h. verbogen werden (der verbogene Zustand ist mit gestrichelten Linien angedeutet).

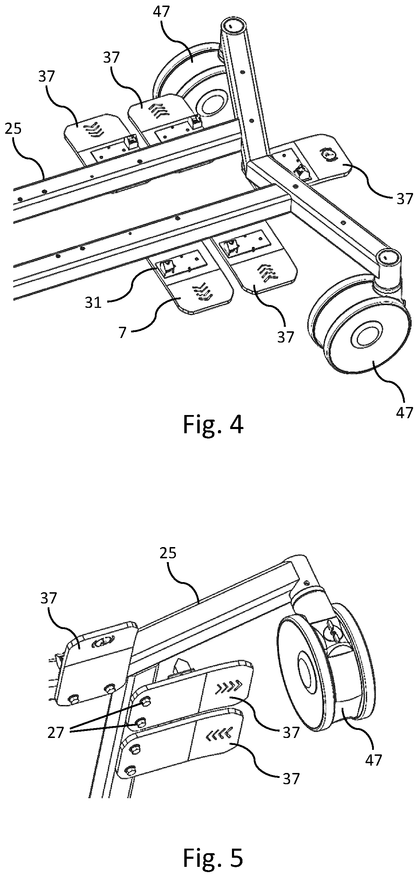

In diesem Beispiel ist das Betätigungselement 7 mit einem Abschnitt eines Fahrgestells 25 (siehe auch Fig. 3 bis Fig. 5) als dem Befestigungsabschnitt 23 über zwei Schrauben 27 verschraubt und durch Herunterdrücken mittels eines Fußes verformbar. Alternativ oder zusätzlich zu den Schrauben 27 kann das Betätigungselement 7 beispielsweise mittels Schweißen, Löten und/oder Kleben am Befestigungsabschnitt 23 befestigt sein.

Wie in Fig. 2 zu sehen, können die beiden Sensorelemente 9a, 9b so angeordnet sein, dass sie eine gedachte gerade Verbindungslinie 29 zwischen den beiden Schrauben 27 überlappen. In diesem Bereich wird das Betätigungselement 7 bei dessen Betätigung in der Regel am stärksten verformt.

Um die Montage bzw. Demontage zu vereinfachen, sind die Signalverarbeitungseinrichtung 13 und die Rückmeldungseinrichtung 19 in diesem Beispiel auf einer gemeinsamen Leiterplatte 31 angeordnet, die über mehrere Abstandhalter 33 auf der Oberseite des Betätigungselements 7 befestigt ist. Die Signalverarbeitungseinrichtung 13 und die Rückmeldungseinrichtung 19 befinden sich dabei in einem Spalt 35 zwischen der Leiterplatte 31 und dem Betätigungselement 7 und sind so gut vor Umwelteinflüssen geschützt. Zusätzlich kann der Spalt 35 staub- und/oder wasserdicht verschlossen sein.

Wie in Fig. 1 angedeutet, kann die Rückmeldungseinrichtung 19 beispielsweise ausgebildet sein, um den Spalt 35 zu beleuchten, wobei ein Teil des ausgesandten Lichts nach außen dringen kann, sodass dem Anwender eine optische Rückmeldung bei der Betätigung des Betätigungselements 7 gegeben werden kann.

Wie in Fig. 3 bis Fig. 5 gezeigt, kann die Bedienvorrichtung 1 eine oder mehrere weitere Betätigungselemente 37 mit jeweils einem weiteren Sensor zum Erfassen einer Verformung des jeweiligen weiteren Betätigungselements umfassen. Die weiteren Betätigungselemente 37 und die weiteren Sensoren können analog zum vorstehend beschriebenen Betätigungselement 7 und dessen Sensor 9 ausgebildet sein.

In diesem Fall kann unter Verwendung eines weiteren Sensorsignals, das eine mittels eines der weiteren Sensoren erfasste Verformung von einem der weiteren Betätigungselemente bei dessen Betätigung anzeigt, ein weiteres Steuersignal zum Steuern der Aktorik 15 erzeugt werden, beispielsweise durch eine entsprechende weitere Signalverarbeitungseinrichtung analog zur vorstehend beschriebenen Signalverarbeitungseinrichtung 13.

Das weitere Steuersignal kann beispielsweise unter zusätzlicher Verwendung des Sensorsignals 11 oder von mindestens einem der Sensorsignale 11a, 11b erzeugt werden. Umgekehrt ist es möglich, dass das Steuersignal 17 unter zusätzlicher Verwendung des weiteren Sensorsignals oder der weiteren Sensorsignale erzeugt wird. Dies ermöglicht es, eine versehentliche gleichzeitige Betätigung der verschiedenen Betätigungselemente 7, 37 zu erkennen.

Wie in Fig. 3 gezeigt, kann der Inkubator 3 eine Inkubatorkammer 39 zum Aufnehmen des Früh- oder Neugeborenen 5 umfassen. In diesem Fall kann die Aktorik 15 beispielsweise eine elektrisch steuerbare Kammerverstelleinrichtung 41 zum Bewegen der Inkubatorkammer 39 (als Ganzes) und/oder eine elektrisch steuerbare Haubenverstelleinrichtung 43 zum Öffnen und/oder Schließen einer verschieb- und/oder schwenkbar gelagerten Haube 45 der Inkubatorkammer 39 umfassen.

Das Fahrgestell 25 kann mehrere Rollen 47 zum Verfahren des Inkubators 3 auf einem Boden umfassen. In diesem Fall kann die Aktorik 15 beispielsweise eine elektrisch steuerbare Antriebs- und/oder Brems- und/oder Lenkeinrichtung 49 zum Antreiben und/oder Bremsen und/oder Lenken von mindestens einer der Rollen 47 umfassen.

Zusätzlich oder alternativ kann der Inkubator 3 eine verstellbare Liegefläche 51 für das Früh- oder Neugeborene 5 umfassen. In diesem Fall kann die Aktorik 15 eine elektrisch steuerbare Liegeflächenverstelleinrichtung 53 zum Verstellen der Liegefläche 51, beispielsweise von deren Neigung und/oder Höhe, umfassen. Die Liegefläche 51 und die Inkubatorkammer 39 können beispielsweise unabhängig voneinander verstellbar sein. Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs der durch die Ansprüche definierten Gegenstände zu verstehen.

### Liste der Bezugszeichen

- 1: Bedienvorrichtung
- 3: medizintechnisches Gerät, Inkubator
- 5: Früh- oder Neugeborenes
- 7: Betätigungselement
- 9: Sensor
- 9a: erstes Sensorelement
- 9b: zweites Sensorelement
- 11: Sensorsignal
- 11a: erstes Sensorsignal
- 11b: zweites Sensorsignal
- 13: Signalverarbeitungseinrichtung
- 15: Aktorik
- 17: Steuersignal
- 19: Rückmeldungseinrichtung
- 21: zusätzliches Steuersignal
- 23: Befestigungsabschnitt
- 25: Fahrgestell
- 27: Schraube
- 29: Verbindungslinie
- 31: Leiterplatte
- 33: Abstandhalter
- 35: Spalt
- 37: weiteres Betätigungselement
- 39: Inkubatorkammer
- 41: Kammerverstelleinrichtung
- 43: Haubenverstelleinrichtung
- 45: Haube
- 47: Rolle
- 49: Antriebs- und/oder Brems- und/oder Lenkeinrichtung
- 51: Liegefläche
- 53: Liegeflächenverstelleinrichtung

## Patentansprüche

1. Verfahren zum Steuern einer Aktorik (15) eines medizintechnischen Geräts (3), wobei das medizintechnische Gerät (3) zusätzlich zur Aktorik (15) eine Bedienvorrichtung (1) zum Bedienen des medizintechnischen Geräts (3) umfasst, wobei die Bedienvorrichtung (1) ein mittels eines Fußes und/oder einer Hand betätigbares Betätigungselement (7) und einen Sensor (9) zum Erfassen einer Verformung des Betätigungselements (7) umfasst, wobei das Verfahren umfasst:
Empfangen eines Sensorsignals (11), das eine mittels des Sensors (9) erfasste Verformung des Betätigungselements (7) bei dessen Betätigung anzeigt;
Erzeugen eines Steuersignals (17) zum Steuern der Aktorik (15) unter Verwendung des Sensorsignals (11).

2. Verfahren nach Anspruch 1,
wobei die Bedienvorrichtung (1) ferner eine Rückmeldungseinrichtung (19) zum Erzeugen einer akustischen und/oder optischen und/oder haptischen Rückmeldung für einen Anwender des medizintechnischen Geräts (3) umfasst;
wobei das Verfahren ferner umfasst:
Erzeugen eines zusätzlichen Steuersignals (21) zum Steuern der Rückmeldungseinrichtung (19) unter Verwendung des Sensorsignals (11) und/oder des Steuersignals (17).

3. Verfahren nach einem der vorhergehenden Ansprüche,
wobei die erfasste Verformung einen erfassten Verformungsgrad umfasst und das Steuersignal (17) abhängig vom erfassten Verformungsgrad erzeugt wird; und/oder
wobei die erfasste Verformung eine erfasste Verformungsrichtung umfasst und das Steuersignal (17) abhängig von der erfassten Verformungsrichtung erzeugt wird; und/oder
wobei der Sensor (9) einen mit zumindest einem Abschnitt des Betätigungselements (7) mechanisch gekoppelten Dehnungsmessstreifen (9a, 9b) umfasst und das Sensorsignal (11) eine an den Anschlüssen des Dehnungsmessstreifens (9a, 9b) anliegende elektrische Spannung und/oder einen zwischen den Anschlüssen des Dehnungsmessstreifens (9a, 9b) fließenden elektrischen Strom als die erfasste Verformung anzeigt.

4. Verfahren nach Anspruch 3,
wobei eine Abweichung einer Amplitude der Spannung und/oder des Stroms von einem Schwellenwert bestimmt wird und das Steuersignal (17) abhängig von der Abweichung erzeugt wird.

5. Verfahren nach einem der vorhergehenden Ansprüche,
wobei das Empfangen des Sensorsignals (11) umfasst:
Empfangen eines ersten Sensorsignals (11a), das eine mittels eines ersten Sensorelements (9a) des Sensors (9) erfasste Verformung eines ersten Abschnitts des Betätigungselements (7) bei dessen Betätigung anzeigt;
Empfangen eines zweiten Sensorsignals (11b), das eine mittels eines zweiten Sensorelements (9b) des Sensors (9) erfasste Verformung eines vom ersten Abschnitt abweichenden zweiten Abschnitts des Betätigungselements (7) bei dessen Betätigung anzeigt;
wobei das Steuersignal (17) unter Verwendung des ersten Sensorsignals (11a) und/oder des zweiten Sensorsignals (11b) erzeugt wird.

6. Verfahren nach einem der vorhergehenden Ansprüche,
wobei die Bedienvorrichtung (1) ferner ein mittels eines Fußes und/oder einer Hand betätigbares weiteres Betätigungselement (37) und einen weiteren Sensor zum Erfassen einer Verformung des weiteren Betätigungselements (37) umfasst;
wobei das Verfahren ferner umfasst:
Empfangen eines weiteren Sensorsignals, das eine mittels des weiteren Sensors erfasste Verformung des weiteren Betätigungselements (37) bei dessen Betätigung anzeigt;
Erzeugen eines weiteren Steuersignals zum Steuern der Aktorik (15) unter Verwendung des weiteren Sensorsignals oder unter Verwendung des Sensorsignals (11) und des weiteren Sensorsignals.

7. Signalverarbeitungseinrichtung (13), umfassend Mittel, die konfiguriert sind, um das Verfahren nach einem der vorhergehenden Ansprüche auszuführen.

8. Bedienvorrichtung (1) zum Bedienen eines medizintechnischen Geräts (3), wobei die Bedienvorrichtung (1) umfasst:
ein mittels eines Fußes und/oder einer Hand betätigbares Betätigungselement (7);
einen Sensor (9), der konfiguriert ist, um eine Verformung des Betätigungselements (7) bei dessen Betätigung zu erfassen und ein die erfasste Verformung des Betätigungselements (7) anzeigendes Sensorsignal (11) zu erzeugen;
eine Signalverarbeitungseinrichtung (13) nach Anspruch 7.

9. Bedienvorrichtung (1) nach Anspruch 8,
wobei das Betätigungselement (7) plattenartig und/oder aus Metall ausgebildet ist; und/oder
wobei das Betätigungselement (7) an seinem ersten Ende starr mit einem Befestigungsabschnitt (23) des medizintechnischen Geräts (3) verbindbar ist und durch Aufbringen einer definierten Biegekraft auf sein zweites, freies Ende betätigbar ist.

10. Bedienvorrichtung (1) nach Anspruch 8 oder 9,
wobei mindestens eine Komponente (13, 19) der Bedienvorrichtung (1) auf einer Leiterplatte (31) angeordnet ist, wobei die Leiterplatte (31) am Betätigungselement (7) befestigt ist.

11. Bedienvorrichtung (1) nach Anspruch 10,
wobei die Leiterplatte (31) mittels mindestens eines Abstandhalters (33) am Betätigungselement (7) befestigt ist, sodass die Leiterplatte (31) und das Betätigungselement (7) durch einen Spalt (35) voneinander getrennt sind, wobei die mindestens eine auf der Leiterplatte (31) angeordnete Komponente (13, 19) im Spalt (35) angeordnet ist.

12. Bedienvorrichtung (1) nach Anspruch 11,
wobei der Spalt (35) zusätzlich abgedichtet ist, um die mindestens eine im Spalt (35) angeordnete Komponente (13, 19) vor Umwelteinflüssen zu schützen.

13. Bedienvorrichtung (1) nach einem der Ansprüche 10 bis 12, ferner umfassend:
eine Rückmeldungseinrichtung (19) zum Erzeugen einer akustischen und/oder optischen und/oder haptischen Rückmeldung für einen Anwender des medizintechnischen Geräts (3);
wobei die Mittel der Signalverarbeitungseinrichtung (13) konfiguriert sind, um das Verfahren nach Anspruch 2 auszuführen;
wobei die mindestens eine auf der Leiterplatte (31) angeordnete Komponente (13, 19) mindestens eine Komponente der Rückmeldungseinrichtung (19) umfasst.

14. Medizintechnisches Gerät (3), umfassend:
eine Aktorik (15);
eine Bedienvorrichtung (1) nach einem der Ansprüche 8 bis 13.

15. Medizintechnisches Gerät (3) nach Anspruch 14,
wobei das medizintechnische Gerät (3) ein Wärmetherapiegerät ist; und/oder
wobei das medizintechnische Gerät (3) ferner eine Liegefläche (51) für einen Patienten (5) umfasst, wobei die Aktorik (15) eine durch die Signalverarbeitungseinrichtung (13) der Bedienvorrichtung (1) steuerbare Liegeflächenverstelleinrichtung (53) zum Bewegen der Liegefläche (51) umfasst; und/oder
wobei das medizintechnische Gerät (3) ferner eine Inkubatorkammer (39) zum Aufnehmen eines Früh- oder Neugeborenen (5) umfasst, wobei die Aktorik (15) mindestens eine der folgenden Einrichtungen, die durch die Signalverarbeitungseinrichtung (13) der Bedienvorrichtung (1) steuerbar sind, umfasst: eine Kammerverstelleinrichtung (41) zum Bewegen der Inkubatorkammer (39); eine Haubenverstelleinrichtung (43) zum Bewegen einer Haube (45) der Inkubatorkammer (39); und/oder
wobei das medizintechnische Gerät (3) ferner ein Fahrgestell (25) mit mehreren Rollen (47) zum Verfahren des medizintechnischen Geräts (3) umfasst, wobei die Aktorik (15) mindestens eine der folgenden Einrichtungen, die durch die Signalverarbeitungseinrichtung (13) der Bedienvorrichtung (1) steuerbar sind, umfasst: eine Antriebseinrichtung (49) zum Antreiben von mindestens einer der Rollen (47); eine Bremseinrichtung (49) zum Bremsen von mindestens einer der Rollen (47); eine Lenkeinrichtung (49) zum Lenken von mindestens einer der Rollen (47).
